# FASCICULE DE BREVET EUROPEEN

(11) **EP 0 666 076 B1**
(45) Date de publication et mention de la délivrance du brevet: **23.12.1998**
(21) Numéro de dépôt: 94402619.4
(22) Date de dépôt: 15.12.1994
(51) Int. Cl.: A61K 7/48, A61K 7/06, B65D 47/44, B65B 31/00, B65D 83/14, B65D 81/26, B65D 81/30

(54) **Emulsion eau dans huile contenant du rétinol**
Wasser in Öl Emulsion enthaltend Retinol
Water in oil emulsion containing retinol

(30) Priorité: 30.12.1993 FR 9315862
(43) Date de publication de la demande: 09.08.1995
(60) Demande divisionnaire: 98118997.0
(73) Titulaire: L'OREAL, 75008 Paris (FR)
(72) Inventeur: Collin, Nathalie, Résidence "Les Cariatides", F-92330 Sceaux (FR); Quemin, Eric, F-93420 Villepinte (FR)
(74) Mandataire: Lhoste, Catherine

(56) Documents cités:
- EP-A- 0 206 343
- EP-A- 0 343 444
- EP-A- 0 508 848
- EP-A- 0 521 278
- EP-A- 0 530 862
- WO-A-93/00085
- FR-A- 2 448 488
- FR-A- 2 645 512
- FR-A- 2 666 308
- US-A- 4 485 133
- US-A- 5 092 914

## Description

La présente invention a pour objet des émulsions eau dans huile contenant, de manière stable, du rétinol, destinées en particulier aux domaines cosmétique et/ou dermatologique. L'invention se rapporte aussi à une utilisation de cette émulsion pour le traitement de la peau

Le rétinol est connu pour ses effets bénéfiques sur la peau, notamment en application topique.

Le rétinol est, depuis longtemps, utilisé dans le traitement de l'acné. Mais c'est dans le domaine de la correction des dommages induits soit par l'âge, soit par une exposition au soleil trop intensive, que le rétinol s'est révélé extrêmement actif.

Ainsi, les effets du rétinol sur la différenciation cellulaire permettent d'envisager, entre autre, son utilisation pour lutter efficacement contre l'apparition des rides et ridules, contre la sécheresse cutanée, la rugosité et/ou la rigidité de la peau. Son activité dans la régénération des tissus en fait un composé important dans la cicatrisation. Une application répétée de compositions cosmétiques contenant du rétinol permet, entre autre, d'effacer les rides, de lisser la peau, de réparer les petites déchirures de l'épiderme.

Le document EP-508848 décrit une composition pour lutter contre le vieillissement de la peau contenant en association un rétinoïde et une dialkyl- ou trialkylxanthine.
Du fait de ces effets bénéfiques, on cherche depuis très longtemps, à formuler le rétinol dans des compositions cosmétiquement acceptables, sous forme stable pendant au moins plusieurs mois à température ambiante.

Des émulsions utilisables en cosmétique contenant du rétinol sont décrites, en particulier dans le document US-A-4 826 828 et dans le document WO-A-93/00085.

Dans le document US-A-4 826 828, il s'agit d'émulsions eau dans huile contenant le rétinol, une silicone volatile, ainsi qu'un solvant du rétinol et de la silicone volatile. Le solvant préféré est l'éthanol. Or, la demanderesse a montré récemment que le rétinol se dégrade en présence d'éthanol. Pour obtenir l'émulsion, il est enseigné dans ce document US-A-4 826 828 de préparer une solution contenant le rétinol à mélanger au moment de l'emploi, pour éviter toutes dégradations de celui-ci, à une émulsion eau dans huile. En outre, l'emploi d'antioxydant et d'agent chélateur de métaux dans la phase aqueuse est indiqué comme essentiel.

La stabilité de telles compositions, comme l'indique le détenteur du brevet US-A-4 826 828 sur les emballages de ses produits Bioadvance et Bioadvance 2000 décrits dans ce brevet, n'excède pas un mois. Ainsi, la stabilité du rétinol dans ce type de compositions est insuffisante au regard d'une utilisation prolongée, ce qui nécessite un réapprovisionnement rapide, donc coûteux.

Dans le document WO-A-93/00085, il est proposé une stabilisation du rétinol dans les compositions cosmétiques par adjonction à celles-ci d'un complexe stabilisant, comprenant associés, un antioxydant et un agent chélateur d'ions métalliques. Si, toutefois, la stabilité du rétinol semble améliorée dans de telles compositions (60 % du rétinol étant encore présent dans la composition après trois mois de conservation à 40°C), il n'en demeure pas moins que la stabilité relative du rétinol n'est due qu'à la présence, dans la composition, d'une quantité importante d'antioxydants et d'agents chélateurs stabilisants.

De nombreuses recherches ont été menées afin de diminuer au maximum, voir d'éliminer, la présence de tels stabilisants dans les compositions cosmétiques contenant du rétinol, tout en conservant à celui-ci une stabilité dans la composition, acceptable au regard de ses effets et en relation avec une utilisation prolongée de la composition.

De manière surprenante et inattendue, la demanderesse a découvert qu'il est possible de formuler des émulsions eau dans huile, stables dans le temps, contenant du rétinol, destinées à une utilisation topique, par un choix approprié des différents constituants de l'émulsion dans le but de conférer à celle-ci des propriétés physico-chimiques particulières.

La présente invention a donc pour objet une émulsion eau dans huile contenant du rétinol, une phase grasse et une phase aqueuse, caractérisée par le fait que la phase grasse contient, au moins, un solvant organique du rétinol liquide à 20°C et choisi dans le groupe constitué par les alcools gras aliphatiques à chaîne ramifiée en C₁₆-C₂₀, les diesters d'acide dicarboxylique en C₆-C₁₄ et d'alcool isopropylique, les triglycérides d'acide gras en C₆-C₁₈ et leurs mélanges, et que le pH de la phase aqueuse est basique, de façon à stabiliser le rétinol, au moins 2 mois.

Les essais réalisés avec une émulsion selon l'invention ont montré une stabilité du rétinol nettement améliorée par rapport à celle de l'Art antérieur.

L'émulsion selon l'invention a l'avantage de pouvoir contenir encore 82 % de rétinol actif après conservation à 45°C pendant 2 mois.

L'émulsion selon l'invention est parfaitement bien adaptée à une utilisation cosmétique et/ou dermatologique.

Les proportions des différents constituants de l'émulsion selon l'invention sont celles classiquement utilisées dans le domaine cosmétique ou dermatologique et sont fonction de l'application spécifique envisagée.

En outre, dans une réalisation préférentielle, l'émulsion selon l'invention est exempte de tout agent chélateur d'ions métalliques et éventuellement d'agent antioxydant.

En particulier, la phase grasse de l'émulsion selon l'invention peut représenter de 10 % à 50 % du poids total de l'émulsion et de préférence de 20 % à 35 %.

Par ailleurs, la phase aqueuse de l'émulsion selon l'invention peut représenter de 50 % à 90 % du poids total de l'émulsion et de préférence de 65 % à 80 %.

De manière générale, le rétinol est présent dans l'émulsion selon l'invention dans une proportion allant de 0,001 % à 10 %, et de manière préférentielle de 0,01 % à 1 % du poids total de l'émulsion.

Le rétinol utilisé dans l'émulsion selon l'invention peut être du rétinol dans l'une quelconque de ses conformations et en particulier celui sous la forme tout-trans, tel que celui vendu par la Société Fluka sous la dénomination "all-trans-rétinol". Le rétinol tout-trans a la particularité de présenter une activité de rétinoïde et une innocuité supérieure aux autres rétinoïdes.

Le solvant organique du rétinol peut représenter de 0,01 % à 30 % du poids total de l'émulsion selon l'invention, et de préférence de 0,1 % à 10 %.

Le solvant organique liquide à température ambiante (à 20°C par exemple) utilisé dans l'invention est n'importe quel solvant organique susceptible de dissoudre le rétinol tout en lui conservant une bonne stabilité. En particulier, ce solvant est choisi dans le groupe constitué par les alcools gras aliphatiques à chaîne ramifiée en C₁₆-C₂₀, les diesters d'acide dicarboxylique en C₆-C₁₄ et d'alcool isopropylique, les triglycérides d'acide gras en C₆-C₁₈ et leurs mélanges.

Les solvants du type alcools gras à chaîne droite ou ramifiée, alcoxylés, détruisent la stabilité de l'émulsion selon l'invention.

Parmi les alcools gras aliphatiques en C₁₆-C₂₀ utilisables dans l'invention, on peut citer, l'hexyl-2-décanol tel que celui vendu par la Société Condea sous la dénomination "Isofol 16", l'octyldodécanol tel que celui vendu par la Société Henkel sous la dénomination "Eutanol G", et l'alcool isostéarylique tel que celui vendu par la Société Sherex sous la dénomination de "Adol 66".

Parmi les diesters dicarboxyliques en C₆-C₁₄ et d'alcool isopropylique utilisables dans l'invention, on peut citer l'adipate de di-isopropyle tel que celui vendu par la Société ISP sous la dénomination de "Ceraphyl 230".

Parmi les triglycérides d'acides gras en C₆-C₁₈, selon l'invention, on préfère utiliser des triglycérides mixtes des acides capriques/capryliques tels que ceux vendus par la Société Hüls France sous la dénomination de "Miglyol 812".

Dans une réalisation particulière de l'invention, il est possible d'utiliser le mélange de deux ou plusieurs de ces solvants du rétinol.

D'autres constituants conventionnels des compositions cosmétiques, pharmaceutiques ou vétérinaires peuvent être introduits dans les émulsions selon l'invention. La nature de ces ingrédients et leur proportion doit être compatible avec la stabilité recherchée du rétinol dans les émulsions selon l'invention.

La phase grasse de l'émulsion selon l'invention peut contenir un émulsionnant capable de former une émulsion eau dans huile et supportant des pH basiques. Cet émulsionnant est en particulier un émulsionnant siliconé utilisé dans une proportion de 0,5 % à 10 % et de préférence de 1 % à 6 % du poids total de l'émulsion. Parmi les émulsionnants pouvant entrer dans la composition de la phase grasse, on peut citer les alkyl diméthicone copolyols en C₁₀-C₂₂ polyoxyéthylénés tels que le cétyl diméthicone copolyol ou "Abil EM-90" de la Société Goldschmidt, ou le lauryl diméthicone copolyol polyoxyéthyléné et polyoxypropyléné comme le Q2-5200 de Dow Corning, et un mélange des deux.

On peut aussi utiliser les diméthicone copolyols polyoxyéthylénés tel que le SP 1228 de General Electric et également le Q2-3225 C de Dow Corning.

La phase grasse peut également contenir une huile complémentaire inerte vis-à-vis du rétinol, différente de l'émulsionnant que l'on choisit de préférence parmi les huiles minérales ou siliconées.

Parmi les huiles minérales utilisables selon l'invention, on peut citer, entre autre, l'isohexadécane, la paraffine, l'isoparaffine, la vaseline.

Parmi les huiles siliconées utilisables dans l'invention, on peut citer, entre autre, les diméthicones, les diméthiconols, les cyclométhicones telles que la cyclopentadiméthylsiloxane (ou cyclométhicone D5) ou la cyclohexadiméthylsiloxane (ou cyclométhicone D6) ou les alkyl diméthicones et un mélange de certains de ces composés tel que la "gomme Q2-1401" de Dow Corning.

La phase aqueuse de l'émulsion selon l'invention a un pH basique supérieur à 8 et de préférence allant de 8,5 à 9,5. Le pH de la phase aqueuse est obtenu par adjonction de toute base compatible avec la stabilité du rétinol dans l'émulsion selon l'invention et en quantité appropriée pour obtenir le pH recherché de la phase aqueuse. En pratique, on utilisera la base à raison d'une proportion allant de 0,05 % à 0,5 % du poids total de l'émulsion.

Parmi les bases utilisées dans l'invention, on peut citer par exemple la triéthanolamine, l'aminométhylpropanol, l'aminométhylpropanediol, le tris ou aminométhylpropanetriol, la soude, l'arginine, la lysine.

L'émulsion selon l'invention peut contenir, en outre, des additifs hydrophiles ou lipophiles classiquement utilisés dans les compositions cosmétiques ou dermatologiques.

Parmi les additifs hydrophiles utilisés dans l'émulsion selon l'invention, on peut citer les hydratants comme les polyols (le glycérol) et les glycols.

Parmi les additifs lipophiles, on peut citer les gélifiants comme le gel de bentone, par exemple celui vendu sous le nom de "Simagel SI 345" par la Société Biophil, les cires de polyéthylène, comme la cire de polyéthylène AC 617 de Allied Chemical.

L'émulsion selon l'invention contient avantageusement des sels stabilisants d'origine non métallique dans une proportion, par exemple, de 0,3 % à 2 % du poids total de l'émulsion tels, que le chlorure de sodium (NaCl), le chlorure de potassium (KCl).

L'invention se rapporte aussi à une utilisation cosmétique d'une émulsion définie ci-dessus pour le traitement de la peau et des dommages induits par l'âge et/ou le soleil. Elle se rapporte aussi à une utilisation d'une émulsion définie ci-dessus, pour la préparation d'un produit destiné au traitement de la peau, de l'acné et des dommages induits par l'âge et/ou le soleil.

L'invention se rapporte également à un procédé de préparation de l'émulsion selon l'invention telle que définie précédemment.

Cette préparation est effectuée en atmosphère inerte ( azote ou gaz rare tel que l'Argon ) exempte de tout oxygène et en présence de lumière inactinique telle que celle d'une lampe à vapeur de sodium.

On va maintenant décrire un procédé général de préparation des émulsions selon l'invention. Dans un premier temps, les phases grasse (A) et aqueuse (B) constituant l'émulsion sont préparées indépendamment, puis la phase aqueuse (B) est incorporée à la phase grasse (A) sous agitation au Moritz, moyenne vitesse, à température ambiante, dans les conditions normales de préparation de toutes émulsions eau dans huile. Le mélange ainsi formé constitue le support émulsionné dans lequel on va incorporer le rétinol.

Sous une tente à azote, à température ambiante, en salle éclairée par une lampe à vapeur de sodium, on fait dégazer l'ensemble des éléments de l'émulsion selon l'invention afin d'éliminer toute trace d'oxygène et de remplacer celle-ci par de l'azote. Ce dégazage s'effectue pendant au moins deux heures. Quand toute trace d'oxygène est évacuée, (on peut vérifier ceci par utilisation d'un oxymètre), on pèse le rétinol et son solvant, et on les mélange sous agitation magnétique jusqu'à solubilisation totale pour former une phase (C). Puis on incorpore cette phase (C) à l'émulsion par agitation à la pâle pour une bonne homogénéisation. Une fois terminée, l'émulsion est incorporée toujours en atmosphère inerte d'azote et sous lumière de vapeur de sodium dans un conditionnement tel que défini ci-dessus.

Nous allons maintenant, de manière non limitative et à titre d'exemple, donner des compositions de diverses émulsions réalisées selon l'invention.

### Exemple 1- Crème contour des yeux

| | | |
|---|---|---|
| A- | Abil EM-90 | 2 % |
| | Cyclométhicone D5 | 7 % |
| | Simagel Si 345 | 5 % |
| | Miglyol 812 | 7,5 % |
| | Gomme Q2 1401 | 4 % |
| B- | Glycérine | 5 % |
| | Triéthanolamine | 0,2 % |
| | Eau permutée | 61,3 % |
| C- | Isofol 16 | 7,5 % |
| | Rétinol | 0,5 % |

Cette crème appliquée en contour des yeux en cure de 2 à 5 fois par semaine a un effet de lissage des fines ridules du contour de l'oeil ou "pattes d'oie"

### Exemple 2 - Crème Antirides

| | | |
|---|---|---|
| A- | Abil EM-90 | 3 % |
| | Cyclométhicone D5 | 7 % |
| | Simagel Si 345 | 5 % |
| | Mygliol 812 | 11,5 % |
| | Gomme Q2-1401 | 3 % |
| B- | Glycérine | 5 % |
| | Triéthanolamine | 0,2 % |
| | NaCl | 0,7 % |
| | Eau permutée qsp | 100 |
| C- | Isofol 16 | 3 % |
| | Rétinol | 0,18 % |

Cette crème très douce et nourrissante est idéale en application quotidienne sur le visage, le soir, pour un effet anti-rides à long terme.

### Exemple 3 - Lait corporel anti-âge

| | | |
|---|---|---|
| A- | Abil EM-90 | 1 % |
| | Isolan Gi 34 | 5 % |
| | Paraffine liquide | 13 % |
| | Cyclométhicone D5 | 10 % |
| | Gomme Q2-1401 | 4 % |
| | Glycérine | 5 % |
| B- | Triéthanolamine | 0,2 % |
| | NaCl | 0,6 % |
| | Eau permutée qsp | 100 |
| C- | Diisopropyl adipate | 1 % |
| | Rétinol | 0,012 % |

Ce lait corporel souple est un excellent soin corporel anti-âge à condition de l'utiliser quotidiennement sur l'ensemble du corps. Après application, la peau est adoucie et lissée.

### Exemple 4 - Soin réparateur des lèvres

| | | |
|---|---|---|
| A- | Abil EM-90 | 3 % |
| | Simagel Si 345 | 5 % |
| | Cyclométhicone | 7 % |
| | Gomme Q2-1401 | 3 % |
| B- | AMP | 0,12 % |
| | NaCl | 0,7 % |
| | Glycérine | 5 % |
| | Eau permutée qsp | 100 |
| C- | Mygliol 812 | 11 % |
| | Rétinol | 0,9 % |

Cette crème compacte très glissante à l'application constitue un soin nourrissant et réparateur des lèvres abîmées et ridées.

Par ailleurs, il a été réalisé un test de stabilité dans lequel on a comparé une émulsion selon l'invention (exemple 1) et différentes formules dérivées de celles de l'exemple 1, (A,B,C,D,E,F).

### Composition A

| | |
|---|---|
| Phase grasse de l'exemple 1 à laquelle on ajoute : | |
| α-tocophérol | 0,1 % |
| acétate d' α-tocophérol | 1 % |
| palmitate d'ascorbyle | 0,2 % |
| + Phase aqueuse de l'exemple 1 dans laquelle on ajoute : | |
| Desquest 2046 * | 0,2 % |
| et dans laquelle l'eau permutée a été ajustée en conséquence. | |

| | |
|---|---|
| * Sel pentasodique de l'acide éthylène diamine tétra-méthylène phosphonique à 33 % tel que vendu par la Société Monsanto | |

### Composition B

| | |
|---|---|
| Phase grasse de l'exemple 1 à laquelle on ajoute : | |
| Palmitate d'ascorbyle | 0,2 % |
| [(ditertiobutyl-3,5 hydroxy-4) benzylidène]-3 camphre | 0,5 % |
| + Phase aqueuse de la composition A. | |

### Composition C

Phase grasse de l'exemple 1 + phase aqueuse de l'exemple 1 de laquelle on a retiré la triéthanolamine.

### Composition D

Phase grasse de la composition de l'exemple 1 + phase aqueuse de l'exemple 1 + phase C contenant le rétinol dissous dans 0,8 g de polysorbate 20 et dans 6,9 g de cyclohexadiméthylsiloxane.

### Composition E

Phase grasse de l'exemple 1 à laquelle on a ajouté 0,5% d'éthoxyquine pure (Raluquin de la Société Raschig) + phase aqueuse de l'exemple 1.

### Composition F

Composition de l'exemple 1 dans laquelle est incorporé après fabrication l'absorbeur d'oxygène isolé de l'émulsion.

Les tests de stabilité de ces compositions ont été effectués par mesure du pourcentage de dégradation après conservation pendant 1 mois ou 2 mois, soit à 4°C, soit à température ambiante, soit à 45°C. Les résultats sont donnés dans le tableau suivant :

| | 1 MOIS | | | 2 MOIS | | |
|---|---|---|---|---|---|---|
| Compositions | 4°C | Températ. ambiante | 45°C | 4°C | Températ. ambiante | 45°C |
| 1 | 0 | 0 | 19 | 6 | 10 | 33 |
| A | 2 | 5 | 25 | 4 | 12 | 35 |
| B | 0 | 1 | 27 | 2 | 18 | 37 |
| C | 0 | 8 | 44 | 4 | 34 | 72 |
| D | 0 | 1 | 40 | 0 | 14 | 68 |
| E | 0 | 0 | 9 | 0 | 0 | 27 |
| F | 0 | 1 | 6 | 1 | 16 | 18 |

Les résultats de ces tests montrent que l'émulsion selon l'invention (ex.1, F) présente une stabilité du rétinol comparable voire meilleure, à celle contenant un ou plusieurs antioxydants (A, B) comme décrit dans l'Art antérieur alors que des émulsions réalisées avec soit un rétinol dissous dans un solvant autre que ceux sélectionnés dans l'invention (D), soit un rétinol en présence d'une phase aqueuse à un pH inapproprié (C) présentent une moins bonne stabilité. Ce tableau montre la possibilité de stabiliser le rétinol dans des émulsions sans antioxydant, mais montre également que le choix d'un antioxydant particulier (Raluquin, E) introduit dans l'émulsion selon l'invention, peut améliorer la stabilité.

## Revendications

1. Emulsion eau dans huile contenant du rétinol, une phase grasse, une phase aqueuse et un émulsionnant de la phase aqueuse dans la phase grasse, caractérisée par le fait que la phase grasse contient, au moins un solvant organique du rétinol, liquide à 20°C et choisi parmi les alcools gras aliphatiques à chaîne ramifiée en C₁₆-C₂₀, les diesters d'acide dicarboxylique en C₆-C₁₄ et d'alcool isopropylique, les triglycérides d'acide gras en C₆-C₁₈ et leurs mélanges, et que le pH de la phase aqueuse est basique de façon à stabiliser le rétinol au moins deux mois.

2. Emulsion selon la revendication 1, caractérisée par le fait qu'elle est à usage cosmétique ou dermatologique.

3. Emulsion selon l'une quelconque des revendications 1 ou 2, caractérisée par le fait qu'elle est exempte de tout agent chélateur d'ions métalliques.

4. Emulsion selon l'une quelconque des revendications précédentes, caractérisée par le fait que la phase grasse représente de 10 % à 50 % du poids total de l'émulsion.

5. Emulsion selon l'une quelconque des revendications précédentes, caractérisée par le fait que la phase grasse représente de 20 % à 35 % du poids total de l'émulsion.

6. Emulsion selon l'une quelconque des revendications précédentes, caractérisée par le fait que la phase aqueuse représente de 50 % à 90 % du poids total de l'émulsion.

7. Emulsion selon l'une quelconque des revendications précédentes, caractérisée par le fait que la phase aqueuse représente de 65 % à 80 % du poids total de l'émulsion.

8. Emulsion selon l'une quelconque des revendications précédentes, caractérisée par le fait qu'elle contient du rétinol à raison de 0,001 % à 10 % du poids total de l'émulsion.

9. Emulsion selon l'une quelconque des revendications précédentes, caractérisée par le fait qu'elle contient du rétinol à raison de 0,01 % à 1 % du poids total de l'émulsion.

10. Emulsion selon l'une quelconque des revendications précédentes, caractérisée par le fait que le solvant organique représente de 0,01 % à 30 % du poids total de l'émulsion.

11. Emulsion selon l'une quelconque des revendications précédentes, caractérisée par le fait que le solvant organique représente de 0,1 % à 10 % du poids total de l'émulsion.

12. Emulsion selon l'une quelconque des revendications précédentes. caractérisée par le fait que le rétinol est du rétinol tout-trans.

13. Emulsion selon l'une quelconque des revendications précédentes, caractérisée par le fait que l'émulsionnant est un émulsionnant siliconé.

14. Emulsion selon l'une quelconque des revendications précédentes, caractérisée par le fait que l'émulsionnant siliconé est en une proportion de 0,5 % à 10 % du poids total de l'émulsion.

15. Emulsion selon l'une quelconque des revendications précédentes, caractérisée par le fait que l'émulsionnant siliconé est en une proportion de 1 % à 6 % du poids total de l'émulsion.

16. Emulsion selon l'une quelconque des revendications 13 à 15, caractérisée par le fait que l'émulsionnant est choisi parmi les alkyldiméthicone copolyols en C₁₀-C₂₂ polyoxyéthylénés et/ou polyoxypropylénés et leurs mélanges.

17. Emulsion selon l'une quelconque des revendications précédentes, caractérisée par le fait que la phase grasse contient une huile complémentaire inerte vis-à-vis du rétinol.

18. Emulsion selon la revendication précédente, caractérisée par le fait que l'huile complémentaire est choisie parmi les huiles minérales et siliconées.

19. Emulsion selon la revendication précédente, caractérisée par le fait que l'huile est choisie parmi le groupe constitué de l'isohexadecane, l'isoparaffine, la vaseline, la paraffine, les diméthicones, les diméthiconols, les cyclométhicones, les alkyldiméthicones.

20. Emulsion selon l'une des revendications précédentes, caractérisée par le fait que la phase aqueuse a un pH allant de 8,5 à 9,5.

21. Emulsion selon l'une quelconque des revendications précédentes, caractérisée par le fait que la phase aqueuse contient une base choisie parmi la triéthanolamine, l'aminométhylpropanol, l'aminométhylpropanediol, le tris (aminométhylpropanetriol), la soude, l'arginine, la lysine.

22. Emulsion selon l'une quelconque des revendications précédentes, caractérisée par le fait qu'elle contient au moins un additif choisi parmi les polyols, les glycols, les sels stabilisants, le gel de Bentone, les cires lourdes ou polyéthylénées, la vaseline.

23. Emulsion selon l'une quelconque des revendications précédentes, caractérisée par le fait qu'elle contient au moins un sel stabilisant dans une proportion de 0,3 % à 2 % du poids total de l'émulsion.

24. Emulsion selon la revendication précédente, caractérisée par le fait que le sel stabilisant est un sel d'un élément non métallique.

25. Emulsion selon l'une quelconque des revendications précédentes, caractérisée par le fait qu'elle est conditionnée de sorte à ne pas être en contact avec l'oxygène et des éléments métalliques, et à être à l'abri de la lumière.

26. Utilisation cosmétique d'une émulsion selon l'une quelconque des revendications précédentes, pour le traitement de la peau et des dommages induits par l'âge et/ou le soleil.

27. Utilisation d'une émulsion selon l'une quelconque des revendications précédentes, pour la préparation d'un produit destiné au traitement de la peau, de l'acné et des dommages induits par l'âge et/ou le soleil.

28. Procédé de préparation de l'émulsion selon l'une quelconque des revendications 1 à 25, caractérisé par le fait que l'émulsion est préparée sous atmosphère inerte, exempte de tout oxygène, et sous lumière inactinique.

29. Procédé de préparation selon la revendication précédente, caractérisé par le fait que l'atmosphère inerte est constituée par de l'azote ou un gaz rare.

## Claims

1. Water-in-oil emulsion containing retinol, a fatty phase, an aqueous phase and an agent for emulsifying the aqueous phase in the fatty phase, characterized in that the fatty phase contains at least one organic solvent for retinol which is liquid at 20°C and chosen from C₁₆-C₂₀ branched-chain aliphatic fatty alcohols, C₆-C₁₄ dicarboxylic acid diesters of isopropyl alcohol, and C₆-C₁₈ fatty acid triglycerides and mixtures thereof, and in that the pH of the aqueous phase is basic, so as to stabilize the retinol for at least two months.

2. Emulsion according to Claim 1, characterized in that it is for cosmetic or dermatological use.

3. Emulsion according to either of Claims 1 and 2, characterized in that it is free of any metal ion-chelating agent.

4. Emulsion according to any one of the preceding claims, characterized in that the fatty phase represents from 10 % to 50 % of the total weight of the emulsion.

5. Emulsion according to any one of the preceding claims, characterized in that the fatty phase represents from 20 % to 35 % of the total weight of the emulsion.

6. Emulsion according to any one of the preceding claims, characterized in that the aqueous phase represents from 50 % to 90 % of the total weight of the emulsion.

7. Emulsion according to any one of the preceding claims, characterized in that the aqueous phase represents from 65 % to 80 % of the total weight of the emulsion.

8. Emulsion according to any one of the preceding claims, characterized in that it contains retinol in an amount of from 0.001 % to 10 % of the total weight of the emulsion.

9. Emulsion according to any one of the preceding claims, characterized in that it contains retinol in an amount of from 0.01 % to 1 % of the total weight of the emulsion.

10. Emulsion according to any one of the preceding claims, characterized in that the organic solvent represents from 0.01 % to 30 % of the total weight of the emulsion.

11. Emulsion according to any one of the preceding claims, characterized in that the organic solvent represents from 0.1 % to 10 % of the total weight of the emulsion.

12. Emulsion according to any one of the preceding claims, characterized in that the retinol is all-trans retinol.

13. Emulsion according to any one of the preceding claims, characterized in that the emulsifying agent is a silicone-containing emulsifying agent.

14. Emulsion according to any one of the preceding claims, characterized in that the silicone-containing emulsifying agent is in a proportion of from 0.5 % to 10 % of the total weight of the emulsion.

15. Emulsion according to any one of the preceding claims, characterized in that the silicone-containing emulsifying agent is in a proportion of from 1 % to 6 % of the total weight of the emulsion.

16. Emulsion according to any one of Claims 13 to 15, characterized in that the emulsifying agent is chosen from polyoxyethylenated and/or polyoxypropylenated C₁₀-C₂₂ all dimethicone copolyols and mixtures thereof.

17. Emulsion according to any one of the preceding claims, characterized in that the fatty phase contains an additional oil which is inert with respect to retinol.

18. Emulsion according to the preceding claim, characterized in that the additional oil is chosen from mineral oils and silicone-containing oils.

19. Emulsion according to the preceding claim, characterized in that the oil is chosen from the group consisting of isohexadecane, isoparaffin, vaseline, paraffin, dimethicones, dimethiconols, cyclomethicones and alkyldimethicones.

20. Emulsion according to one of the preceding claims, characterized in that the aqueous phase has a pH ranging from 8.5 to 9.5.

21. Emulsion according to any one of the preceding claims, characterized in that the aqueous phase contains a base chosen from triethanolamine, aminomethylpropanol, aminomethylpropanediol, tris (aminomethylpropanetriol), sodium hydroxide, arginine and lysine.

22. Emulsion according to any one of the preceding claims, characterized in that it contains at least one additive chosen from polyols, glycols, stabilizing salts, Bentone gel, heavy or polyethylenated waxes, and vaseline.

23. Emulsion according to any one of the preceding claims, characterized in that it contains at least one stabilizing salt in a proportion of from 0.3 % to 2 % of the total weight of the emulsion.

24. Emulsion according to the preceding claim, characterized in that the stabilizing salt is a salt of a non-metal element.

25. Emulsion according to any one of the preceding claims, characterized in that it is packaged so as not to be in contact with oxygen or metal components, and so that it is protected from light.

26. Cosmetic use of an emulsion according to any one of the preceding claims for treating the skin and for treating damage caused by age and/or the sun.

27. Use of an emulsion according to any one of the preceding claims for the preparation of a product intended for treating the skin, for treating acne and for treating damage caused by age and/or the sun.

28. Process for preparing the emulsion according to any one of Claims 1 to 25, characterized in that the emulsion is prepared under an atmosphere which is inert and free of any oxygen, and under inactinic light.

29. Preparation process according to the preceding claim, characterized in that the inert atmosphere consists of nitrogen or a rare gas.

## Patentansprüche

1. Wasser-in-Öl-Emulsion, die Retinol, eine Fettphase, eine wäßrige Phase und einen Emulgator zum Emulgieren der wäßrigen Phase in der Fettphase enthält, dadurch gekennzeichnet, daß die Fettphase mindestens ein organisches Lösungsmittel für das Retinol enthält, das bei 20 °C flüssig ist und unter aliphatischen Fettalkoholen mit verzweigter C₁₆₋₂₀-Kette, den Diestern aus einer C₆₋₁₄-Dicarbonsäure und Isopropanol, den Triglyceriden einer C₆₋₁₈-Fettsäure und deren Gemischen ausgewählt ist, und daß der pH-Wert der wäßrigen Phase basisch ist, um das Retinol für eine Dauer von mindestens zwei Monaten zu stabilisieren.

2. Emulsion nach Anspruch 1, dadurch gekennzeichnet, daß sie für die kosmetische oder dermatologische Verwendung vorgesehen ist.

3. Emulsion nach einem der Ansprüche 1 und 2, dadurch gekennzeichnet, daß sie keinen Chelatbildner für Metallionen enthält.

4. Emulsion nach einem der vorhergehenden Ansprüche, dadurch gekennzeichnet, daß die Fettphase 10 bis 50 % des Gesamtgewichts der Emulsion ausmacht.

5. Emulsion nach einem der vorhergehenden Ansprüche, dadurch gekennzeichnet, daß die Fettphase 20 bis 35 % des Gesamtgewichts der Emulsion ausmacht.

6. Emulsion nach einem der vorhergehenden Ansprüche, dadurch gekennzeichnet, daß die wäßrige Phase 50 bis 90 % des Gesamtgewichts der Emulsion ausmacht.

7. Emulsion nach einem der vorhergehenden Ansprüche, dadurch gekennzeichnet, daß die wäßrige Phase 65 bis 80 % des Gesamtgewichts der Emulsion ausmacht.

8. Emulsion nach einem der vorhergehenden Ansprüche dadurch gekennzeichnet, daß sie Retinol in einem Anteil von 0,001 bis 10 % des Gesamtgewichts der Emulsion enthält.

9. Emulsion nach einem der vorhergehenden Ansprüche, dadurch gekennzeichnet, daß sie Retinol in einem Anteil von 0,01 bis 1 % des Gesamtgewichts der Emulsion enthält.

10. Emulsion nach einem der vorhergehenden Ansprüche, dadurch gekennzeichnet, daß das organische Lösungsmittel 0,01 bis 30 % des Gesamtgewichts der Emulsion ausmacht.

11. Emulsion nach einem der vorhergehenden Ansprüche, dadurch gekennzeichnet, daß das organische Lösungsmittel 0,1 bis 10 % des Gesamtgewichts der Emulsion ausmacht.

12. Emulsion nach einem der vorhergehenden Ansprüche, dadurch gekennzeichnet, daß es sich bei dem Retinol um *all-trans*-Retinol handelt.

13. Emulsion nach einem der vorhergehenden Ansprüche, dadurch gekennzeichnet, daß der Emulgator ein Silicon-Emulgator ist.

14. Emulsion nach einem der vorhergehenden Ansprüche, dadurch gekennzeichnet, daß der Silicon-Emulgator in einem Anteil von 0,5 bis 10 % des Gesamtgewichts der Emulsion enthalten ist.

15. Emulsion nach einem der vorhergehenden Ansprüche, dadurch gekennzeichnet, daß der Silicon-Emulgator in einem Anteil von 1 bis 6 % des Gesamtgewichts der Emulsion enthalten ist.

16. Emulsion nach einem der Ansprüche 13 bis 15, dadurch gekennzeichnet, daß der Emulgator unter polyethoxylierten und/oder polypropoxylierten Alkyldimethiconcopolyolen mit 10 bis 22 Kohlenstoffatomen und deren Gemischen ausgewählt ist.

17. Emulsion nach einem der vorhergehenden Ansprüche, dadurch gekennzeichnet, daß die Fettphase ein zusätzliches, gegenüber Retinol inertes Öl enthält.

18. Emulsion nach dem vorhergehenden Anspruch, dadurch gekennzeichnet, daß das zusätzliche Öl unter Mineralölen und Siliconölen ausgewählt ist.

19. Emulsion nach dem vorhergehenden Anspruch, dadurch gekennzeichnet, daß das Öl unter Isohexadecan, Isoparaffin, Vaseline, Paraffin, Dimethiconen, Dimethiconolen, Cyclomethiconen, Alkyldimethiconen ausgewählt ist.

20. Emulsion nach einem der vorhergehenden Ansprüche, dadurch gekennzeichnet, daß die wäßrige Phase einen pH-Wert im Bereich von 8,5 bis 9,5 aufweist.

21. Emulsion nach einem der vorhergehenden Ansprüche, dadurch gekennzeichnet, daß die wäßrige Phase eine Base enthält, die unter Triethanolamin, Aminomethylpropanol, Aminomethylpropandiol, Tris (Aminomethylpropantriol), Natriumhydroxid, Arginin, Lysin ausgewählt ist.

22. Emulsion nach einem der vorhergehenden Ansprüche, dadurch gekennzeichnet, daß sie mindestens einen Zusatz enthält, der unter Polyolen, Glykolen, stabilisierenden Salzen, Benton-Gel, schweren bzw. hochsiedenden Wachsen und Polyethylen-Wachsen, Vaseline ausgewählt ist.

23. Emulsion nach einem der vorhergehenden Ansprüche, dadurch gekennzeichnet, daß sie mindestens ein stabilisierendes Salz in einem Anteil von 0,3 bis 2 % des Gesamtgewichts der Emulsion enthält.

24. Emulsion nach dem vorhergehenden Anspruch, dadurch gekennzeichnet, daß das stabilisierende Salz ein Salz eines nichtmetallischen Elements ist.

25. Emulsion nach einem der vorhergehenden Ansprüche, dadurch gekennzeichnet, daß es so verpackt ist, daß es nicht mit Sauerstoff und metallischen Elementen in Kontakt ist und vor Licht geschützt ist.

26. Kosmetische Verwendung einer Emulsion nach einem der vorhergehenden Ansprüche zur Behandlung der Haut und der durch das Altern und/oder die Sonne hervorgerufenen Schäden.

27. Verwendung einer Emulsion nach einem der vorhergehenden Ansprüche für die Herstellung eines Produkts, das für die Behandlung der Haut, von Akne und der durch das Altern und/oder die Sonne hervorgerufene Schäden vorgesehen ist.

28. Verfahren zur Herstellung der Emulsion nach einem der Ansprüche 1 bis 25, dadurch gekennzeichnet, daß die Emulsion unten Inertgasatmosphäre, die keinen Sauerstoff enthält, und unter nicht aktinischem Licht hergestellt wird.

29. Herstellungsverfahren nach dem vorhergehenden Anspruch, dadurch gekennzeichnet, daß die Inertgasatmosphäre aus Stickstoff oder einem Edelgas besteht.
